# EUROPEAN PATENT APPLICATION

(11) **EP 3 440 994 A1**
(43) Date of publication of application: **13.02.2019**
(21) Application number: 17185733.7
(22) Date of filing: 10.08.2017
(51) Int. Cl.: A61B 5/0205, A61B 5/08, A61B 5/00

(54) **APPARATUS AND METHOD FOR MONITORING SLEEP APNEA**

(30) Priority: 08.08.2017 KR 20170100550
(71) Applicant: HB Tech Future Technology Research Center, Cheonan-si, Chungcheongnam-do 31075 (KR)
(72) Inventor: Lee, Un Hak, 31471 Chungcheongnam-do (KR); Kim, Si Kyung, 31204 Chungcheongnam-do (KR); Seo, Bong Gyun, 31102 Chungcheongnam-do (KR); Choi, Chul Hyung, 31089 Chungcheongnam-do (KR)
(74) Representative: Petraz, Gilberto Luigi

(57) **Abstract**

An apnea monitoring apparatus (100) may include a motion detection module, an apnea detection module (220), a processor (200), and a communication module (240). The motion detection module is attached to a body of a sleeping person and detects a motion of the sleeping person. The apnea detection module is attached to the body of the sleeping person and detects apnea of the sleeping person. The processor recognizes a sleeping state of the sleeping person and drives the apnea detection module when there is no motion detected by the motion detection module for a predetermined time. Also, the processor generates apnea information when the apnea of the sleeping person is detected by the apnea detection module. The communication module transmits the apnea information to an electronic device. The electronic device stimulates the sleeping person, based on the apnea information transmitted from the apnea monitoring apparatus.

## Description

### [Technical Field]

The present invention relates to an apparatus and method for monitoring a breathing state of a sleeping person and stimulating the sleeping person in an apnea state to a breathing state.

### [Background Art]

Sleep apnea is a sleep disorder characterized by pauses in breathing or periods of shallow breathing during sleep. Each pause can last for a few seconds to a few minutes and they happen many times a night. Clinically, when a pause in breathing continues ten seconds or more during sleep and when such pauses happen more than five times per hour or happen more than thirty times during seven hours of sleep, this may be diagnosed as sleep apnea. A person with sleep apnea can suffer chronic fatigue and also have a higher risk of developing hypertension, stroke, and the like.

It is difficult for a general person to judge sleep apnea by himself. Normally, in order to monitor a breathing state of a sleeping person and diagnose sleep apnea, it is necessary to visit a hospital troublesomely.

### [Disclosure]

### [Technical Problem]

Therefore, there is a need of an apparatus for detecting sleep apnea in daily life without a visit of a hospital.

Furthermore, it is necessary to implement an apparatus that can stimulate a sleeping person in an apnea state to a breathing state.

### [Technical Solution]

According to various embodiments of the present invention, an apnea monitoring apparatus may comprise a motion detection module attached to a body of a sleeping person and configured to detect a motion of the sleeping person; an apnea detection module attached to the body of the sleeping person and configured to detect apnea of the sleeping person; a processor configured to recognize a sleeping state of the sleeping person and drive the apnea detection module when there is no motion detected by the motion detection module for a predetermined time, and to generate apnea information when the apnea of the sleeping person is detected by the apnea detection module; and a communication module configured to transmit the apnea information to an electronic device. The electronic device may be configured to stimulate the sleeping person, based on the apnea information transmitted from the apnea monitoring apparatus.

According to various embodiments of the present invention, a method for monitoring sleep apnea may comprise steps of recognizing a sleeping state of a sleeping person when there is no motion detected for a predetermined time by analyzing an output of a motion detection sensor attached to the sleeping person; detecting a breathing state, based on an electrocardiogram (ECG) and chest impedance of the sleeping person in the sleeping state; determining the breathing state of the sleeping person, based on the ECG and the chest impedance; and when the breathing state of the sleeping person is determined as an apnea state, generating apnea information of the sleeping person and transmitting the apnea information to an electronic device. The electronic device may stimulate the sleeping person, based on the received apnea information.

### [Advantageous Effects]

According to various embodiments of the present invention, the sleeping state of the sleeping person may be monitored using the apnea monitoring apparatus attached to the sleeping person. If it is determined that the sleeping person is in the apnea state, the sleeping person may be changed to the breathing state by the stimulating apparatus. The apnea reaction system according to various embodiments of the present invention may be configured as a small mobile apnea monitoring device that is easy to adhere to the sleeping person, and may determine and stimulate the apnea state of the sleeping person by collecting and analyzing the ECG and the breathing rate (chest impedance) of the sleeping person.

### [Description of Drawings]

FIG. 1 is a block diagram illustrating an apnea reaction system according to various embodiments of the present invention.
FIG. 2 is a block diagram illustrating an apnea monitoring apparatus of an apnea reaction system according to various embodiments of the present invention.
FIG. 3 is a block diagram illustrating an electronic device of an apnea reaction system according to various embodiments of the present invention.
FIGS. 4A and 4B are block diagrams illustrating an apnea detection module of an apnea monitoring apparatus according to various embodiments of the present invention.
FIG. 5A and 5B are diagrams illustrating examples of determining a breathing state of a sleeping person on the basis of R-R intervals detected by an electrocardiogram detection module.
FIG. 6A and 6B are diagrams illustrating examples of determining a breathing state of a sleeping person on the basis of a breathing rate detected by a breathing rate detection module.
FIG. 7 is a flow diagram illustrating the operation of an apnea reaction system according to various embodiments of the present invention.
FIG. 8 is a flow diagram illustrating the operation of an apnea monitoring apparatus according to various embodiments of the present invention.
FIG. 9 is a flow diagram illustrating the operation of an apnea monitoring apparatus for determining a breathing state of a sleeping person by detecting an electrocardiogram and a breathing rate.
FIG. 10 is a flow diagram illustrating the operation of an apnea monitoring apparatus for determining a breathing state of a sleeping person by sequentially detecting an electrocardiogram and a breathing rate.
FIG. 11 is a flow diagram illustrating the operation of an apnea monitoring apparatus for analyzing an electrocardiographic value.
FIG. 12 is a flow diagram illustrating the operation of an apnea monitoring apparatus for analyzing a breathing rate value.

### [Mode for Invention]

Hereinafter, embodiments of the present invention will be described in detail with reference to the accompanying drawings.

In describing the embodiments, descriptions of techniques which are well known in the art to which the present invention belongs and which are not directly related to the present invention will not be described or illustrated in detail. This is to avoid obscuring the subject matter of the present invention.

For the same reason, some elements are exaggerated, omitted or schematically shown in the accompanying drawings. Also, the size of each element does not entirely reflect the actual size. In the drawings, the same or corresponding elements are denoted by the same reference numerals.

The advantages and features of the present invention and the manner of achieving them will become apparent with reference to the embodiments described in detail below with reference to the accompanying drawings. The present invention may, however, be embodied in many different forms and should not be construed as limited to the embodiments set forth herein. Rather, these embodiments are provided so that this disclosure will be thorough and complete, and will fully convey the scope of the invention to those skilled in the art. To fully disclose the scope of the invention to those skilled in the art, and the invention is only defined by the scope of the claims.

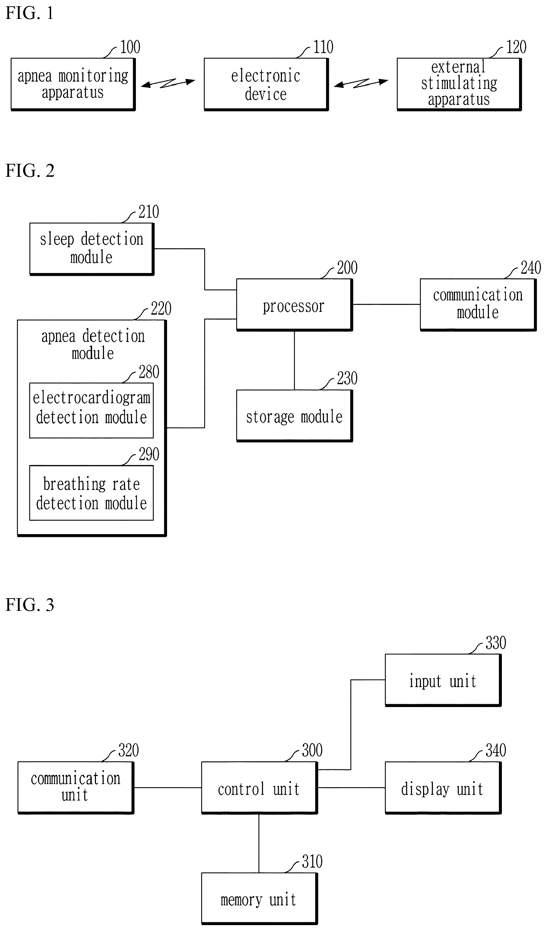

FIG. 1 is a block diagram illustrating an apnea reaction system according to various embodiments of the present invention.

Referring to FIG. 1, the apnea reaction system may include an apnea monitoring apparatus 100, an electronic device 110, and an external stimulating apparatus 120.

The apnea monitoring apparatus 100 may be attached to a body of a sleeping person and may perform functions of monitoring a sleeping state of the sleeping person and analyzing a breathing state of the sleeping person. Namely, the apnea monitoring apparatus 100 may determine whether the sleeping person is in a sleeping state, by detecting a motion of the sleeping person, and also analyze a breathing state of the sleeping person when the sleeping person is in the sleeping state. If the breathing state is determined to be apnea, the apnea monitoring apparatus 100 may generate apnea information and then transmit it to the electronic device 100.

The electronic device 110 may perform an operation for awakening the sleeping person when the apnea information is received from the apnea monitoring apparatus 100. For example, the electronic device 110 may indicate an apnea state through a display unit and also alarm the apnea state through a speaker.

The electronic device 110 according to various embodiments of the present invention may be a smart phone, a tablet personal computer (tablet PC), a mobile phone, a video phone, a desktop PC, a laptop PC, a netbook computer, a workstation, a server, a personal digital assistant (PDA), a portable multimedia player (PMP), an MP3 player, a mobile medical device, a digital camera, or a wearable device. The wearable device may be an accessory-type device (e.g., a watch, a ring, a bracelet, an anklet, a necklace, glasses, a contact lens, or a head-mounted device (HMD)), a fabric or clothes-integrated device (e.g., electronic clothes), a body-attached device (e.g., a skin pad or tattoo), or a bio-implantable device (e.g., an implantable circuit).

In some embodiments, the electronic device 110 may be a home appliance, which may be, for example, a television, a digital video disc (DVD) player, an audio player, a refrigerator, an air conditioner, a vacuum cleaner, an oven, a microwave oven, a washing machine, an air cleaner, a set-top box, a home automation control panel, or a security control panel. In some embodiments, the electronic device 110 may be an internet-of-things (IoT) device (e.g., a lighting device, a thermostat, a heater, a boiler, or an air conditioner). In some embodiments, the electronic device 110 may be a furniture, a bed, or a part of a building or structure.

In various embodiments, the electronic device 110 may be a combination of the above-listed devices. The electronic device 110 according to embodiments of the present invention is not limited to the above-mentioned devices, and may include a new electronic device or any equivalent to be developed in the future.

When the apnea information is received from the apnea monitoring apparatus 100, the electronic device 110 such as a smart phone or a PC may transmit a control signal to the external stimulating apparatus 120 to stimulate the sleeping person. The external stimulating apparatus 120 may be a home appliance, a lighting device, an air conditioner, a bed control device, or the like. For example, if the external stimulating apparatus 120 is a lighting device, the lighting device may be turned on to illuminate the lighting when the control signal is received. If the external stimulating apparatus 120 is an air conditioner, the air conditioner may control the room temperature when the control signal is received. If the external stimulating apparatus 120 is a home appliance, the home appliance may output an audio signal when the control signal is received. If the external stimulating apparatus 120 is a bed control device, the bed control device may move (e.g., tilt, shake, etc.) the bed when the control signal is received.

If the electronic device 110 is a home appliance, a bed, a lighting device, or an air conditioner having the ability to stimulate the sleeping person, the external stimulating apparatus 120 may be omitted. Also, even when the electronic device 110 is a smart phone, a PC, or any equivalent, the external stimulating apparatus 120 may be omitted. In this case, the electronic device 110 may externally indicate an apnea state through the display unit and/or the speaker thereof so as to stimulate the sleeping person. In addition, if the electronic device 110 is a smart phone, a PC, or any equivalent, and if the external stimulating apparatus 120 is used, the electronic device 110 may transmit the control signal to the external stimulating apparatus 120, when the apnea information is received, and may also indicate an apnea state through the display unit and/or the speaker thereof. Then the external stimulating apparatus 120 may be driven to stimulate the sleeping person in response to the control signal received from the electronic device 110.

FIG. 2 is a block diagram illustrating an apnea monitoring apparatus of an apnea reaction system according to various embodiments of the present invention.

Referring to FIG. 2, the apnea monitoring apparatus (e.g., 100 in FIG. 1) may include a processor 200, a sleep detection module 210, an apnea detection module 220, a storage module 230, and a communication module 240. The apnea detection module 220 may include an electrocardiogram detection module 280 and a breathing rate detection module 290.

The sleep detection module 210 may detect the motion of a user while the apnea monitoring apparatus is attached to the user. The sleep detection module 210 may include a motion sensor and detect a motion of a sleeping person through the motion sensor. The motion sensor may be an acceleration sensor and/or a gyro sensor.

The apnea detection module 220 may be driven when the sleeping person is in a sleeping state, and may detect a breathing state of the sleeping person in the sleeping state. As mentioned above, the apnea detection module 220 may include the electrocardiogram detection module 280 and the breathing rate detection module 290. The electrocardiogram detection module 280 may detect the electrocardiogram (hereinafter, also referred to as ECG) of the sleeping person in the sleeping state. The breathing rate detection module 290 may detect the breathing rate of the sleeping person in the sleeping state. For example, the breathing rate detection module 290 may detect the inhalation and exhalation of the sleeping person, based on the chest impedance, and the breathing rate may be defined as the ratio of inhalation to exhalation.

The processor 200 may predetermine reference values for determining the sleeping state of the sleeping person and reference apnea values. People's sleeping states and breathing states may vary. For example, the sleeping state and the breathing state may be varied depending on gender (i.e., male and female), age (e.g., child, adult, the old, etc.), and individual characteristics. Therefore, in order to determine the apnea state during the sleep of the sleeping person, the processor 200 may calculate and store reference motion values for the motion during the sleep (e.g., reference values for motions until the user of the apnea monitoring apparatus enters in the sleeping state from the non-sleeping state). In addition, the processor 200 may measure the ECG and the breathing rate when the user performs normal respiration in the sleeping state, and may store them as a reference ECG value and a reference breathing rate value, respectively.

The processor 200 may determine the sleeping state and the breathing state when it is detected that the apnea monitoring apparatus is attached to the user, and may also generate and transmit the apnea information to the electronic device 110 when it is determined that the state of the user (i.e., the sleeping person) is the apnea state. Specifically, when the apnea monitoring apparatus is mounted on the user, the processor 200 may recognize it through the sleep detection module 210. Then the processor 200 may analyze the output of the sleep detection module 210 and thereby detect the motion of the user. If no motion is detected for a given time, the processor 200 may determine that the user is in the sleeping state.

In the sleeping state, the processor 200 may analyze the breathing state of the sleeping person, based on the output of the apnea detection module 220. For example, the processor 200 may analyze the breathing state of the sleeping person by comparing the output of the ECG detection module 280 with the reference ECG value. Also, the processor 200 may analyze the breathing state of the sleeping person by comparing the output of the breathing rate detection module 290 with the reference breathing rate value. The breathing rate detection module 290 may detect the chest impedance of the sleeping person, and the processor 200 may determine the breathing rate by measuring the ratio of inhalation to exhalation, based on the detected chest impedance. Then the processor 200 may compare the determined breathing rate with the reference breathing rate value so as to analyze the breathing state.

For example, when both the ECG and the breathing rate exceed the range of the reference ECG value and the reference breathing rate vale in the sleeping state, the processor 200 may determine the state of the sleeping person as the apnea state. In another example, when any one of the ECG and the breathing rate exceeds the range of a corresponding reference value in the sleeping state, the processor 200 may determine the state of the sleeping person as the apnea state.

For example, the processor 200 may analyze the breathing state of the sleeping person by inputting in parallel the outputs of the ECG detection module 280 and the breathing rate detection module 290 in the sleeping state. Alternatively, the processor 200 may analyze the ECG and the breathing rate in a serial form in the sleeping state. In the latter case, the processor 200 may analyze first the output of the ECG detection module 280 (or the breathing rate detection module 290) and then, if the ECG (or the breathing rate) exceeds the reference value, analyze the output of the breathing rate detection module 290 (or the ECG detection module 280). Namely, the processor 200 may determine that the sleeping person is in the normal breathing state when the ECG (or the breathing rate) satisfies the range of the reference value, and if the ECG (or the breathing rate) fails to satisfy the reference value range, the processor 200 may analyze the output of the breathing rate (or the ECG) of the sleeping person.

The processor 200 may store, in the storage module 230, information about the breathing state (such as the ECG and the breathing rate) of the sleeping person analyzed in the sleeping state. The storage module 230 may store the apnea information read by the processor 200. For example, the apnea information may include some or all of the occurrence time of apnea, the end time of apnea, the total time of apnea interval, the number of times of apnea, and the like. The storage module 230 may include a volatile memory and/or a nonvolatile memory. The storage module 230 may store instructions or data (e.g., apnea information) related to other elements of the apnea monitoring apparatus.

When it is determined that the sleeping person is in the apnea state, the processor 200 may generate the apnea information and transmit the generated apnea information to the electronic device 110 through the communication module 240. For example, the communication module 240 may be a short-range wireless communication module. Specifically, the communication module 240 may include at least one of a wireless fidelity (WiFi) module, a Bluetooth module, a Bluetooth low energy (BLE) module, a near field communication (NFC) module, a magnetic stripe transmission (MST) module, and a global navigation satellite system (GNSS) module.

As described above, the apnea monitoring apparatus having the configuration as shown in FIG. 2 may monitor the sleeping state of the sleeping person and may also monitor the breathing state of the sleeping person by detecting the ECG and/or the breathing rate (chest impedance) of the sleeping person in the sleeping state. If it is determined that the sleeping person is in the apnea state, the apnea monitoring apparatus may store the result therein and also transmit the result to the electronic device to awake the sleeping person from the sleep.

FIG. 3 is a block diagram illustrating an electronic device of an apnea reaction system according to various embodiments of the present invention.

Referring to FIG. 3, the electronic device (e.g., 110 in FIG. 1) may include a control unit 300, a memory unit 310, a communication unit 320, an input unit 330, a display unit 340, and any other essential or optional element (not shown). The electronic device may be, for example, a smart phone or a PC. In another example, the electronic device may be a device (e.g., a home appliance, a lighting device, an air conditioner, a bed control device, etc.) capable of directly stimulating the sleeping state of the sleeping person. If the electronic device has the ability to stimulate the sleeping state of the sleeping person directly, the external stimulating apparatus (e.g., 120 in FIG. 1) may be omitted.

The memory unit 310 may store programs for executing an application for monitoring sleep and breathing states according to various embodiments. Also, the memory unit 310 may store sleeping and breathing state information of the sleeping person that occurs while the application is executed. The control unit 300 may control a plurality of hardware or software components connected to the control unit 300 by driving an operating system or an application program.

The communication unit 320 may include a cellular communication module and/or a short-range communication module. The cellular communication module may use at least one of long term evolution (LTE), LTE advanced (LTE), code division multiple access (CDMA), wideband CDMA (WCDMA), universal mobile telecommunications system (UMTS), and global system for mobile communications (GSM) technologies. The short-range communication module may use at least one of WiFi, Bluetooth, BLE, Zigbee, NFC, MST, radio frequency (RF), and body area network (BAN) technologies.

The input unit 330 may include a microphone, a keyboard, and/or a keypad, and may generate commands and/or key data. The display unit 340 may further include a speaker to output the apnea information of the user audibly as well as visually. The display unit 340 may include a speaker and/or a lamp to output a sound and/or output a flashing light so as to stimulate the sleeping person in the apnea state under the control of the control unit 300.

The electronic device and/or the external stimulating apparatus may include the short-range communication module (e.g., Bluetooth or BLE), and may have the speaker or vibration function capable of giving an alarm and warning to the sleeping person in the apnea state. Additionally, the electronic device may provide a display or user interface (UI) function for a user's monitoring. The electronic device may be connected to the apnea monitoring apparatus through the short-range communication module, and have functions of displaying monitored data in a graph or any other graphical form and stimulating the sleeping person when receiving the apnea information.

In the apnea monitoring system according to various embodiments of the present invention, the apnea monitoring apparatus may determine the sleeping state, based on the motion of the sleeping person, and monitor the sleeping state of the sleeping person, based on the ECG and/or the breathing rate (chest impedance). If it is determined that the sleeping person is in the apnea state, the apnea monitoring apparatus may notify this to the external stimulating apparatus to stimulate the sleeping person to breathe. The apnea monitoring apparatus may be configured as a small device that can be attached to the body of the sleeping person.

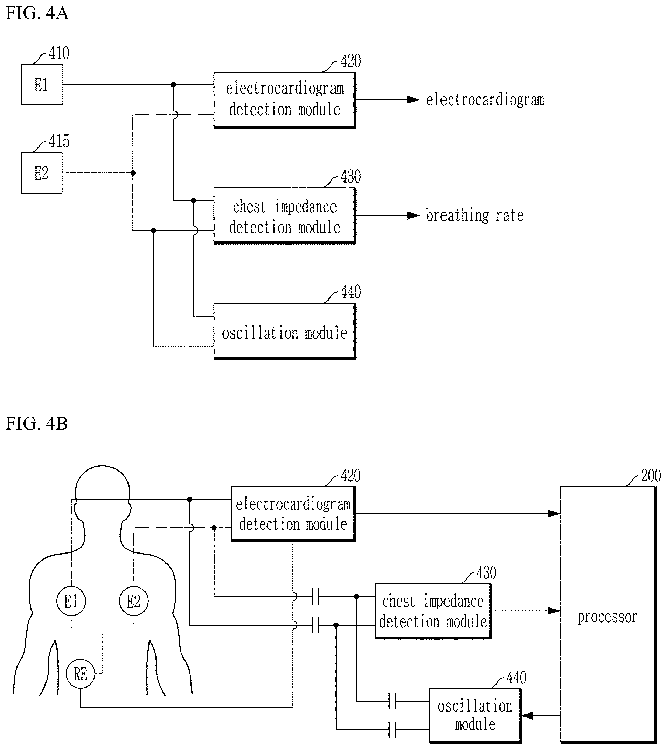

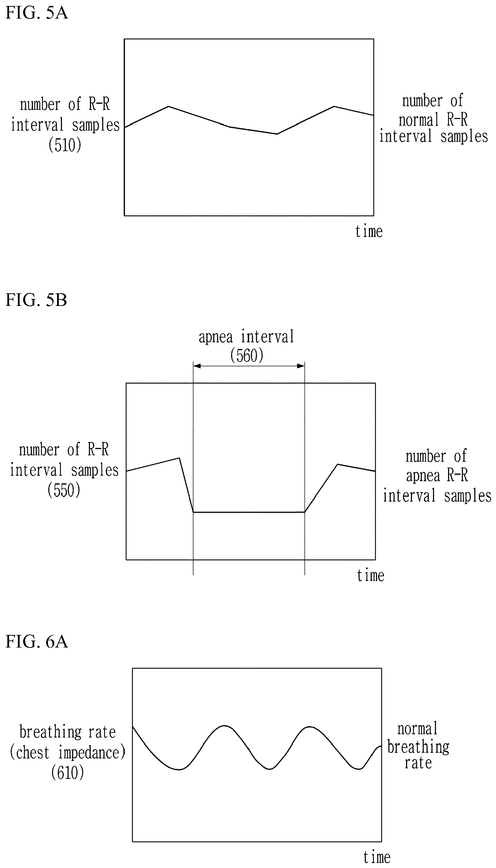

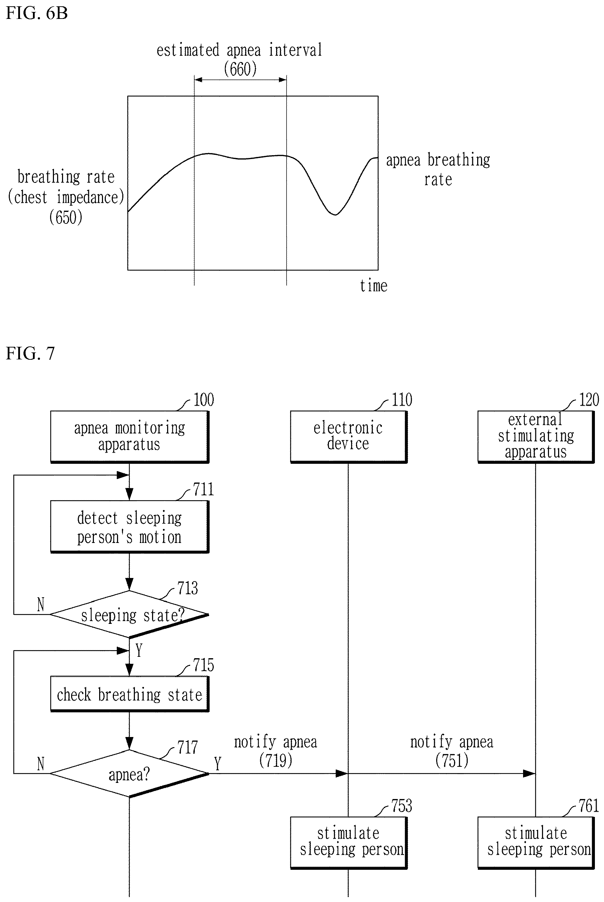

FIGS. 4A and 4B are block diagrams illustrating an apnea detection module (e.g., 220 in FIG. 2) of an apnea monitoring apparatus (e.g., 100 in FIG. 1) according to various embodiments of the present invention. FIG. 5A and 5B are diagrams illustrating examples of determining a breathing state of a sleeping person on the basis of R-R intervals detected by an electrocardiogram detection module (e.g., 280 in FIG. 2). FIG. 6A and 6B are diagrams illustrating examples of determining a breathing state of a sleeping person on the basis of a breathing rate detected by a breathing rate detection module (e.g., 290 in FIG. 2).

Referring to FIGS. 4A and 4B, in order to observe the state of the sleeping person noninvasively, first and second electrodes 410 and 415 may be attached to the body of the sleeping person and measure a biological signal of the sleeping person. Here, the biological signal may be an electrocardiogram (ECG) and a chest impedance. The ECG is a curve measured on the body surface from electrical currents associated with heart muscle activity. When the blood expelled by the contraction of the heart flows along the artery and then flows back into the heart through the vein by the expansion of the heart, the first electrode 410 and the second electrode 415 may detect electrical signals generated by the contraction and expansion of the heart. An ECG detection module 420 may receive the detected electrical signals from the first and second electrodes 410 and 415 and thereby detect the ECG (e.g., R-R interval).

The sleeping person can breathe with inhalation and exhalation, which cause the contraction and expansion of the chest. The chest impedance may be varied according to the volume change in the chest during the contraction and expansion of the chest. A sinusoidal signal may be applied to the body to measure the chest impedance. An oscillation module 440 may apply oscillation signals having a predetermined current (e.g., several mV) at a predetermined frequency to the first and second electrodes 410 and 415. A chest impedance detection module 430 may detect the oscillation signals through the first and second electrodes 410 and 415. At this time, the oscillation signals may be changed by the inhalation and exhalation of the sleeping person. In case of a normal breathing, the time period of inhalation and exhalation (e.g., the breathing rate) may have the same (or similar) ratio. However, if the sleeping person snores or is in the apnea state, the time period of inhalation and exhalation may be varied. The oscillation module 440 may apply oscillation signals of a high-frequency AC constant current through a plurality of electrodes attached to the chest of the sleeping person. The chest impedance detection module 430 may generate impedance information by measuring variations of voltage with respect to variations of chest volume according to breathing of the sleeping person. Here, the impedance information may be a voltage value or an impedance value. That is, the chest impedance detection module 430 may detect the breathing rate of the inhalation and exhalation by detecting the oscillation signals changed by the breathing of the sleeping person. In FIG. 4A, a combination of the oscillation module 440 and the chest impedance detection module 430 may correspond to the breathing rate detection module (e.g., 290 in FIG. 2).

The electrodes (or electrode sensors) attached to the sleeping person are at least two (E1, E2) and may be three or more including a reference potential electrode (RE) as shown in FIG. 4B. Here, E1 and E2 may denote the first electrode 410 and the second electrode 415, respectively. For example, the first electrode (E1) and the second electrode (E2) may be attached to both arms (i.e., right arm (RA) and left arm (LA)), respectively, and the reference potential electrode (RE) may be attached to one leg (e.g., right leg (RL)). Alternatively, the first and second electrodes (E1, E2) may be attached to both sides of the chest. The electrodes (or electrode sensors) may be a consumable adhesive pad or an absorber-type terminal.

The ECG may be a curve of a potential difference that occurs with heartbeat as the heart contracts. The ECG detection module 420 detects the R-R interval of the ECG. Here, 'R' is a symbol of a specific ECG waveform and corresponds to one of five waveforms of 'P-Q-R-S-T'. One feature of the R wave is a greater amplitude than that of the other waves. Therefore, the R wave is easier to detect than the other waves and is used to measure intervals between ECG symbols. The R-R interval may be represented as a time or the number of samples. Data may be reprocessed by accumulating the R-R intervals at a given time period (e.g., 10 seconds). If the obtained data indicates a relatively low state for a certain time compared with the R-R interval in the normal state, it may be estimated as apnea. For example, a reference numeral 510 in FIG. 5A indicates the number of samples of the R-R interval. When the sleeping person normally breathes in the sleeping state, the ECG detection module 420 may detect sample values of the R-R interval as shown in FIG. 5A. However, if the sleeping person is sleeping in the apnea state, the ECG detection module 420 may detect the number of samples of the R-R interval as indicated by a reference numeral 550 in FIG. 5B. In this case, a lower interval 560 may be estimated as the sleep apnea interval.

The processor 200 may calculate the average of the R-R intervals when the detected ECG value is inputted. For example, an average value may be calculated for one minute or for any other specific time. After calculating the average value of the ECG, the processor 200 may compare the detected ECG value with the reference ECG value. If the time when the detected ECG value is lower than the reference ECG value exceeds a predetermined time, it may be estimated as the sleep apnea.

The chest impedance detection module 430 may include an amplification unit, a filter unit, and an A/D converter. When the oscillation module 440 applies a high-frequency AC constant current (e.g., 100 µA / 80 kHz) to the electrodes attached to the chest of the examinee, the chest impedance detection module 430 may amplify signals caused by variations of chest volume according to breathing of the sleeping person, filter the amplified signals, and convert the filtered signals into digital data. Therefore, the chest impedance detection module 430 may detect variations of voltage with respect to variations of chest volume according to breathing of the sleeping person.

For example, a plurality of electrodes for applying a high-frequency AC constant current may be attached to a plurality of portions of the chest of the examinee so as to form a closed circuit including the body of the sleeping person. When the examinee breathes while the high-frequency AC constant current is applied to the examinee's chest through the electrodes, the volume within the chest changes and this variation of chest volume may be detected as a biological signal through the electrodes. The amplification and filter units of the chest impedance detection module 430 may amplify the biological signal detected through the electrodes to a predetermined size and then remove a noise signal. The biological signal from which the noise signal is removed may be impedance breathing amount information (e.g., analog voltage information or impedance information), and then may be converted into digital data by the A/D converter.

The chest impedance detection module 430 may detect the breathing rate (chest impedance) of the sleeping person. As shown in FIG. 6A, the normal breathing rate may indicate similar sizes of inhalation and exhalation. In FIG. 6A, a reference numeral 610 may indicate a chest impedance value (voltage value), and the detected breathing rate has a constant cycle. However, if the sleeping person is in the apnea state, one of inhalation and exhalation may have a relatively long interval, and the chest impedance value (voltage value) detected by the chest impedance detection module 430 may have an irregular form as indicated by a reference numeral 650 in FIG. 6B.

When the detected breathing rate (i.e., the chest impedance value) is inputted, the processor 200 may calculate the time of the detected breathing rate lower than the average value and the time of the detected breathing rate greater than the average value by a predetermined time unit. Namely, for an accurate measurement, the average value of the breathing rate may be used. When the ratio of the calculated times (e.g., the ratio of inhalation to exhalation time) exceeds the range of the reference breathing rate, the processor 200 may estimate that the breathing state of the sleeping person is the apnea state.

As shown in FIGS. 4A and 4B, the apnea detection module (e.g., 220 in FIG. 2) may include an electrode sensor unit 410 and 415, the ECG detection module 420, the breathing rate detection module (e.g. the chest impedance detection module 430 and the oscillation module 440). The electrode sensor unit may be attached to the human body. Each detection module may receive a signal (e.g., about several mV) from the electrode sensor unit through a cable, and output digital data amplified, filtered and converted from the received signal. In this case, the signal outputted from the electrode sensor unit may include the ECG and/or the breathing rate (e.g., chest impedance). In order to amplify the signal of about several mV, the detection module (e.g., the ECG detection module and the chest impedance detection module) may use a differential amplification method having a strong noise characteristic. The breathing rate may be measured through the amplitude of a signal between the first and second electrodes (e.g., a signal passing through the chest) when a clock having a constant frequency is applied. For this, a normal OP-AMP or amplifier, an ADC, a clock generator, or a front-end IC that integrates all of them may be used.

The apnea reaction system according to various embodiments of the present invention may use the motion sensor for monitoring the sleeping state of the sleeping person, and also use the electrode sensor attached to the body (e.g., the chest) of the sleeping person so as to monitor the ECG signal and the breathing rate (e.g., chest impedance). The system may receive ECG data and breathing rate (chest impedance) data having predetermined resolution and sample frequency (e.g., 24 bit resolution and 250 Hz sample frequency) through corresponding detection modules, respectively. Further, the system may receive data related to the motion of the sleeping person through the motion detection module. Here, such data may be downscaled as needed, and the digital filter may include a high pass filter (HPF), a low pass filter (LPF), and the like.

The apnea monitoring apparatus may monitor the sleeping state of the sleeping person before monitoring the ECG and the breathing rate. The motion detection module may measure the motion of the sleeping person by accumulating data detected by the motion sensor, and thereby determine whether the sleeping person is in the sleeping state. For example, such data may be accumulated for one minute or for any other selected time. If it is determined that the sleeping person is in the sleeping state, the apnea monitoring apparatus may detect the R-R interval of the ECG. The R wave is one of five waveforms of 'P-Q-R-S-T', and may have a greater amplitude than that of the other waves. The R-R interval used to measure intervals between ECG symbols may be represented as a time or the number of samples. The apnea monitoring apparatus may reprocess the R-R intervals in units of a given time (e.g., 10 seconds). If the detected R-R interval continues in a relatively low state for a certain time compared with the R-R interval in the normal state, the apnea monitoring apparatus may estimate that this state is the apnea state. Also, the apnea monitoring apparatus may analyze the breathing rate of the sleeping person, based on the detected chest impedance value. The normal breathing rate has similar sizes of inhalation and exhalation. In case of sleep apnea, the interval of inhalation or exhalation becomes relatively long, so the sleep apnea may be estimated through analysis of the intervals of inhalation and exhalation.

If it is determined that the sleeping person is in the apnea state, the apnea monitoring apparatus may generate and store apnea information and transmit the apnea information to the electronic device through the communication module. The apnea information may include the occurrence time of apnea, the end time of apnea, the total time of apnea interval, the number of times of apnea, and/or the like. When the electronic device receives the apnea information, the electronic device may display the received information in the form of graph or text via the UI, and may also output an alarm to the user if any apnea detection message is included. If the electronic device itself includes a function of stimulating the sleeping person, the electronic device may perform the stimulating function to allow the sleeping person to breathe in the sleeping state. If the electronic device does not include the stimulation function, the electronic device may transmit a control signal for stimulating the sleeping person to the external stimulating apparatus.

FIG. 7 is a flow diagram illustrating the operation of an apnea reaction system according to various embodiments of the present invention.

Referring to FIG. 7, the apnea monitoring apparatus 100 may be mounted on the sleeping person. When such mounting is recognized, the apnea monitoring apparatus 100 may detect the motion of the sleeping person by analyzing the output of the motion sensor at step 711. If a detected motion value is smaller than a predetermined reference value, the apnea monitoring apparatus 100 may recognize at step 713 that the sleeping person is in the sleeping state, and then may check the breathing state of the sleeping person at step 715. At this time, the breathing state of the sleeping person may be analyzed, based on the ECG and/or chest impedance of the sleeping person. Namely, the apnea monitoring apparatus 100 may analyze the breathing state of the sleeping person by comparing the detected ECG and/or chest impedance with the corresponding reference value(s). If the value of the detected ECG and/or chest impedance exceeds the range of the reference value, the apnea monitoring apparatus 100 may determine at step 717 that the sleeping person is in the apnea state. Then, at step 719, the apnea monitoring apparatus 100 may generate and store apnea information and transmit it to the electronic device 110 via the communication module.

The electronic device 110 may be an apparatus (e.g., a home appliance, an air conditioner, a lighting device, and/or a bed control device) for stimulating the sleeping state of the sleeping person, or a device (e.g., a smart phone or a PC) capable of controlling the external stimulating apparatus 120 (e.g., a home appliance, an air conditioner, a lighting device, and/or a bed control device) for stimulating the sleeping state of the sleeping person. If the electronic device 110 is a device capable of controlling the external stimulating apparatus 120, the electronic device 110 may alert the apnea state of the sleeping person through the display unit at step 753 and also transmit the apnea information to the external stimulating apparatus 120 at step 751 when the apnea information is received. Then, at step 761, the external stimulating apparatus 120 may stimulate the sleeping person by performing a given function, based on the apnea information received from the electronic device 110. Here, one way of stimulating the sleeping person may be a manner of moving the sleeping person so that the sleeping person in the apnea state can breathe. If the electronic device 110 includes the stimulating function, the electronic device 110 may skip the operation of step 751 and perform the stimulating function at step 753.

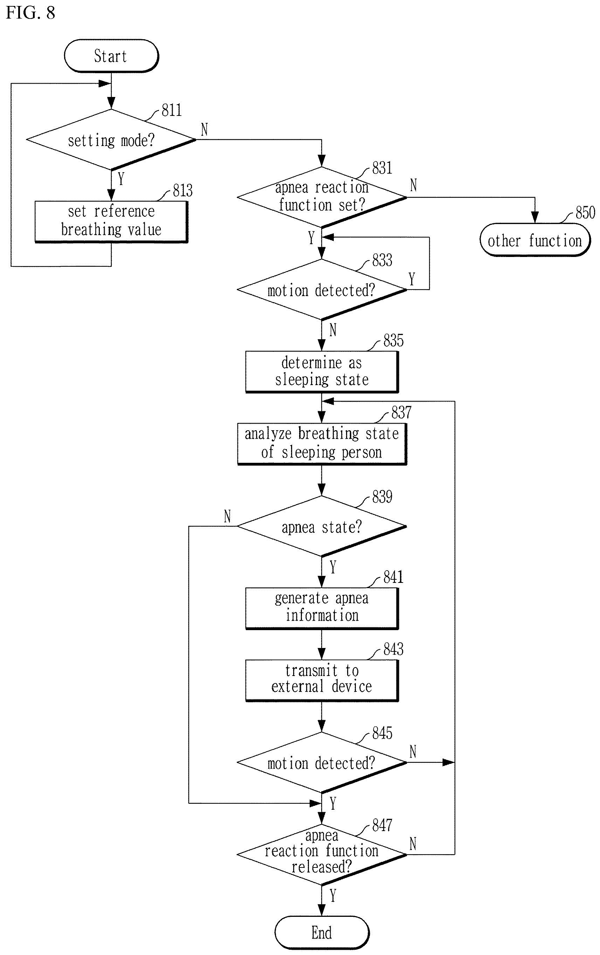

FIG. 8 is a flow diagram illustrating the operation of an apnea monitoring apparatus according to various embodiments of the present invention.

Referring to FIG. 8, people's sleeping and breathing forms may vary. The apnea monitoring apparatus may set the reference values of motion and breathing suitable for each sleeping person. Namely, the apnea monitoring apparatus may have a setting mode. If the setting mode is selected, the apnea monitoring apparatus may recognize the selection of the setting mode at step 811 and then display items for setting the reference values at step 813. If the user selects the displayed item, the apnea monitoring apparatus may measure the selected item and then set a measured result as a reference value at step 813. Here, the items may be a motion value for determining the sleeping state of the sleeping person, an ECG value and/or chest impedance value for determining the breathing state of the sleeping person, and the like. The apnea monitoring apparatus may also download and store the reference values of such items. In this case, the apnea monitoring apparatus may skip the operations of steps 811 and 813.

When the apnea monitoring apparatus is attached to the body of the sleeping person, the apnea monitoring apparatus may recognize at step 831 that the apnea reaction function is set. For example, when the electrode sensor is attached to the body, the apnea monitoring apparatus may recognize attachment and set the apnea reaction function. Also, the apnea monitoring apparatus may include an on/off switch. In this case, when the switch is turned on, the apnea monitoring apparatus may recognize it and set the apnea reaction function.

When the apnea reaction function is set, the apnea monitoring apparatus may determine the sleeping state by analyzing the motion of the user. That is, if the motion of the user is detected to be greater than a predetermined reference value, the apnea monitoring apparatus may determine that the sleeping person is in the non-sleeping state. If the motion of the sleeping person has a motion value smaller than the predetermined reference value, the apnea monitoring apparatus may recognize it at step 833 and determine at step 835 that the sleeping person is in the sleeping state.

In the sleeping state, the apnea monitoring apparatus may analyze the breathing state of the sleeping person at step 837. The breathing state may be determined, based on the detected value of the ECG and/or chest impedance of the sleeping person. The ECG detected in the apnea state may have a lower value than the reference ECG value detected in the normal breathing state. Also, the breathing rate (i.e., the ratio of inhalation to exhalation) detected in the apnea state may exceed the range of the breathing rate in the normal breathing state. The apnea monitoring apparatus may determine the breathing state of the user by comparing the detected breathing values (e.g., the ECG and chest impedance values) with the corresponding reference values. If the detected breathing value exceeds the range of the reference value, the apnea monitoring apparatus may recognize at step 839 that the sleeping person is in the apnea state. Then, at step 841, the apnea monitoring apparatus may generate and store apnea information and transmits the apnea information to the electronic device through the communication module.

In such a case, the electronic device or the external stimulating apparatus may stimulate the sleeping person to breathe. One way of stimulation is to induce breathing of the sleeping person by stimulating the sleeping person to move. If the sleeping person is moved by stimulation, the apnea monitoring apparatus may recognize this at step 845 and may check at step 847 whether the apnea reaction function is released. If the apnea reaction function is not released, the apnea monitoring apparatus may perform again step 837 to analyze the breathing state of the sleeping person.

The sleeping person may not be moved in response to stimulation. That is, the sleeping person in the sleeping state may not react to stimulation. In this case, the apnea monitoring apparatus may recognize this at step 845 and return to step 837 to analyze the breathing state of the sleeping person. Therefore, if the sleeping person maintains the apnea state without any reaction, the apnea monitoring apparatus may continuously stimulate the sleeping person so that the sleeping person can breathe. If the sleeping person changes from the apnea state to the normal breathing state, the apnea monitoring apparatus may recognize this at step 839 and perform step 847.

If the sleeping person awakes from the sleeping state and then removes the attached electrode sensor or turns off the switch, the apnea monitoring apparatus may recognize the release of the apnea reaction function at step 847 and terminate the process.

The apnea monitoring apparatus may determine the breathing state of the sleeping person by detecting both the ECG and the breathing rate. In this case, there are two approaches. One is a parallel approach in which the apnea monitoring apparatus detects the ECG and the breathing rate independently (i.e., in parallel) to determine the apnea state. The other is a serial approach in which the apnea monitoring apparatus detects first the ECG (or the breathing rate) to determine the apnea state, and then, if the apnea state is determined, further detects the breathing rate (or the ECG) to confirm the apnea state.

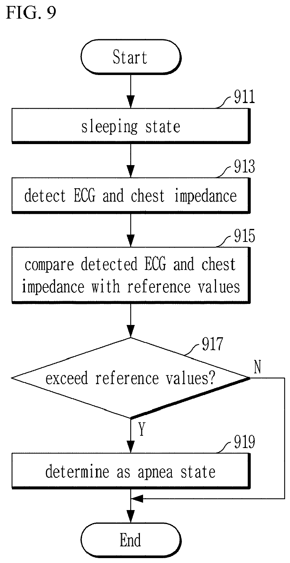

FIG. 9 is a flow diagram illustrating the operation of an apnea monitoring apparatus for determining a breathing state of a sleeping person by detecting an electrocardiogram and a breathing rate.

Referring to FIG. 9, the apnea monitoring apparatus may recognize the sleeping state of the sleeping person at step 911, and may detect the ECG and the chest impedance of the sleeping person at step 913. Then, at step 915, the apnea monitoring apparatus may compare the detected ECG value with the reference ECG value and also compare the detected chest impedance value with the reference chest impedance value. If each of the detected values exceeds the range of the corresponding reference value, the apnea monitoring apparatus may recognize it at step 917 and may set the user's breathing state to the apnea state at step 919. The operation shown in FIG. 8 is an example of the parallel processing approach in which the apnea monitoring apparatus detects both the ECG and the breathing rate.

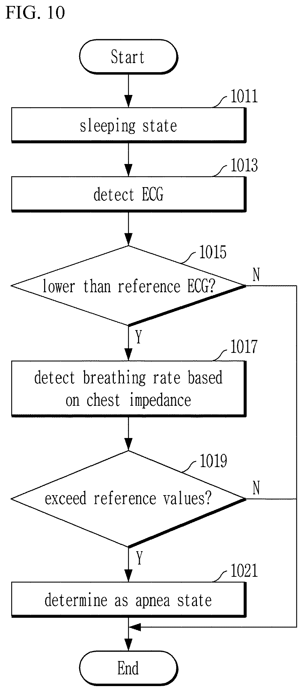

FIG. 10 is a flow diagram illustrating the operation of an apnea monitoring apparatus for determining a breathing state of a sleeping person by sequentially detecting an electrocardiogram and a breathing rate.

Referring to FIG. 10, the apnea monitoring apparatus may recognize the sleeping state of the sleeping person at step 1011, and may detect the ECG of the sleeping person at step 1013. Then the apnea monitoring apparatus may compare the detected ECG value with the reference ECG value at step 1015. If the detected ECG value satisfies the range of the reference ECG value, the apnea monitoring apparatus may determine the breathing state of the sleeping person as a normal breathing state and then terminate the process. However, if the detected ECG value is lower than the reference ECG value, the apnea monitoring apparatus may estimate the apnea state and then detect the breathing rate based on the chest impedance at step 1017. Then the apnea monitoring apparatus may compare the detected breathing rate with the reference breathing rate at step 1019. If the detected breathing rate exceeds the range of the reference breathing rate, the apnea monitoring apparatus may determine at step 1021 that the breathing state of the sleeping person is the apnea state. The operation shown in FIG. 9 is an example of the serial processing approach in which the apnea monitoring apparatus first examines the ECG and then, if the ECG indicates the apnea condition, detects the breathing rate.

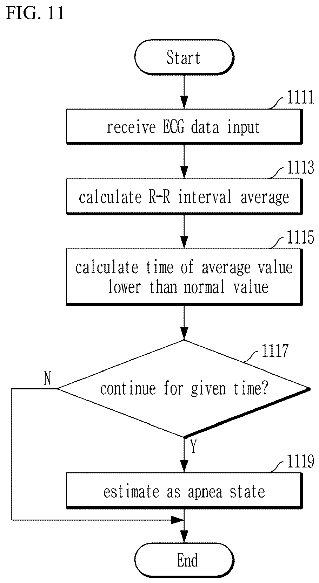

FIG. 11 is a flow diagram illustrating the operation of an apnea monitoring apparatus for analyzing an electrocardiographic value.

Referring to FIG. 11, when ECG values detected by the ECG detection module are inputted at step 1111, the apnea monitoring apparatus may calculate an average value of R-R intervals of the ECG values at step 1113. At this time, the average value may be calculated in a given time period and may be the number of samples. Then, at step 1115, the apnea monitoring apparatus may calculate a time when the average value is lower than a normal value, and may check at step 1117 whether the calculated time continues for a predetermined reference time or longer. If the calculated time is smaller than the predetermined reference time, the apnea monitoring apparatus may determines that the breathing state of the sleeping person is the normal breathing state, and return to step 1111. However, if the calculated time continues for the predetermined reference time or longer, the apnea monitoring apparatus may recognize this at step 1117 and estimate at step 1119 that the breathing state of the sleeping person is the apnea state. Here, the reference ECG value may include a normal average value, a reference time, etc. of R-R interval.

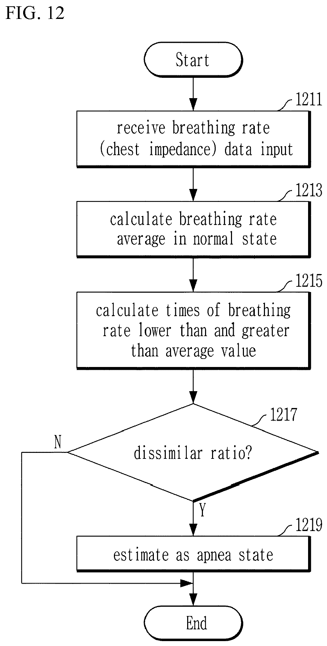

FIG. 12 is a flow diagram illustrating the operation of an apnea monitoring apparatus for analyzing a breathing rate value.

Referring to FIG. 12, when chest impedance values detected by the chest impedance detection module are inputted at step 1211, the apnea monitoring apparatus may calculate an average value of the breathing rate in a normal breathing state at step 1213. At this time, the breathing average value in the normal breathing state may be the reference breathing rate, which may be set by the apnea monitoring apparatus (e.g., performed at steps 811 and 813 in FIG. 8) or downloaded from an external device. Then, at step 1215, the apnea monitoring apparatus may calculate the time of the detected breathing rate lower than the average value and the time of the detected breathing rate greater than the average value. For an accurate measurement, the average value of the breathing rate may be used. Then, at step 1217, the apnea monitoring apparatus may check whether the ratio of the calculated times (e.g., the ratio of inhalation to exhalation times) has a similar value. If so, the normal breathing state is estimated. However, if the ratio does not have a similar value, the apnea monitoring apparatus may recognize this at step 1217 and estimate at step 1219 that the breathing state of the sleeping person is the apnea state. That is, the time ratio of inhalation to exhalation may have the same or similar value in the normal breathing state, whereas in case of the apnea state the inhalation time may be detected to be longer than the exhalation time.

After detecting the ECG and the chest impedance (breathing rate) of the sleeping person, the apnea monitoring apparatus may determine the apnea state or not of the sleeping person by analyzing the detected ECG and the detected chest impedance as shown in FIGS. 11 and 12. If it is determined that the sleeping person is in the apnea state, the apnea monitoring apparatus may transmit the measured value to the electronic device through the communication module (e.g., the BLE module). Then the electronic device may stimulate the sleeping person to breathe.

While the invention has been described in terms of several preferred embodiments, these embodiments are illustrative and not restrictive. It will be understood by those skilled in the art that various changes and modifications may be made without departing from the spirit of the invention and the scope of the appended claims.

## Claims

1. An apnea monitoring apparatus comprising:
a motion detection module attached to a body of a sleeping person and configured to detect a motion of the sleeping person;
an apnea detection module attached to the body of the sleeping person and configured to detect apnea of the sleeping person;
a processor configured to recognize a sleeping state of the sleeping person and drive the apnea detection module when there is no motion detected by the motion detection module for a predetermined time, and to generate apnea information when the apnea of the sleeping person is detected by the apnea detection module; and
a communication module configured to transmit the apnea information to an electronic device,
wherein the electronic device is configured to stimulate the sleeping person, based on the apnea information transmitted from the apnea monitoring apparatus.

2. The apnea monitoring apparatus of claim 1, wherein the apnea detection module includes:
a first electrode and a second electrode separately attached to the body of the sleeping person;
an electrocardiogram (ECG) detection module configured to receive signals from the first and second electrodes and to detect ECG of the sleeping person from the received signals;
an oscillation module configured to apply an oscillation signal to the first and second electrodes; and
a breathing rate detection module configured to detect the oscillation signal between the first and second electrodes and to detect a breathing rate of the sleeping person from the detected oscillation signal.

3. The apnea monitoring apparatus of claim 2, wherein the processor is further configured to:
when the sleeping state is recognized,
compare the ECG detected by the ECG detection module with a reference ECG,
compare the breathing rate detected by the breathing rate detection module with a reference breathing rate, and
when the detected ECG and the detected breathing rate are lower than the reference ECG and the reference breathing rate, respectively, determine that the sleeping person is in an apnea state, and transmits information about the apnea state to the electronic device through the communication module.

4. A method for monitoring sleep apnea, the method comprising steps of:
recognizing a sleeping state of a sleeping person when there is no motion detected for a predetermined time by analyzing an output of a motion detection sensor attached to the sleeping person;
detecting a breathing state, based on an electrocardiogram (ECG) and chest impedance of the sleeping person in the sleeping state;
determining the breathing state of the sleeping person, based on the ECG and the chest impedance; and
when the breathing state of the sleeping person is determined as an apnea state, generating apnea information of the sleeping person and transmitting the apnea information to an electronic device,
wherein the electronic device stimulates the sleeping person, based on the received apnea information.

5. The method of claim 4, wherein the step of detecting the breathing state includes steps of:
receiving signals from first and second electrodes separately attached to a body of the sleeping person and detecting the ECG of the sleeping person from the received signals; and
applying an oscillation signal to the first and second electrodes, detecting the oscillation signal between the first and second electrodes, and detecting a breathing rate of the sleeping person from the detected oscillation signal.

6. The method of claim 4, wherein the step of determining the breathing state of the sleeping person includes steps of:
when the sleeping state is recognized, comparing the detected ECG with a reference ECG and comparing the detected breathing rate with a reference breathing rate; and
when the detected ECG and the detected breathing rate are lower than the reference ECG and the reference breathing rate, respectively, determining that the sleeping person is in the apnea state, and transmitting information about the apnea state to the electronic device.
